# EUROPÄISCHE PATENTSCHRIFT

(11) **EP 2 813 214 B1**
(45) Veröffentlichungstag und Bekanntmachung des Hinweises auf die Patenterteilung: **04.04.2018**
(21) Anmeldenummer: 14153679.7
(22) Anmeldetag: 03.02.2014
(51) Int. Cl.: A61Q 11/00, A61K 8/34, A61K 8/69, A61K 8/86, A61K 9/02

(54) **Flüssigzahncreme mit Aminfluorid(en)**
Liquid dentifrice with aminofluoride(s)
Crème dentaire liquide aux fluorure(s) d'amines

(30) Priorität: 12.06.2013 DE 102013210931
(43) Veröffentlichungstag der Anmeldung: 17.12.2014
(73) Patentinhaber: Henkel AG & Co. KGaA, 40589 Düsseldorf (DE)
(72) Erfinder: Miehlich, Kristin, 42119 Wuppertal (DE); Arians, Daniela, 45239 Essen (DE)

(56) Entgegenhaltungen:
- WO-A1-2006/005211
- DE-A1- 3 715 366

## Beschreibung

Die Erfindung betrifft eine Zahncreme bzw Zahnpasta, die Plaquereduzierung und Fluoriddeposition fördert und zudem den besonderen Erfordernissen bei der Verwendung elektrischer Zahnbürsten Rechnung trägt.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Seit den 50er Jahren werden neben anorganischen Fluoriden in Mund- und Zahnpflege- und - reinigungsmittel auch sogenannte "Aminfluoride" eingesetzt. Aminfluoride, die in der Zahnpflege und - medizin eingesetzt werden, sind kationische Tenside. Sie verfügen über eine lange, hydrophobe Alkylgruppe und ein polares, hydrophiles protoniertes tertiäres Amin mit einem Fluorid als Anion. Aminfluoride hemmen - wie anorganische Fluoride - wichtige bakterielle Enzyme zur Energiegewinnung. Da sie im Vergleich mit anorganischen Fluoriden die Zellmembran sehr leicht durchdringen, erreichen sie auch dort deutlich schneller wirksame Konzentrationen.

Bislang ist der Einsatz von Aminfluoriden auf Zahnpasten beschränkt, da sich diese Verbindungen nur in hochviskose Formulierungen stabil einarbeiten ließen.

Der Einsatz von Aminfluoriden in Produkten zur oralen Hygiene wird in der deutschen Patentanmeldung DE 37 15 366 A1 offenbart. Dort werden Zahncremes mit Olaflur beschrieben, die durch Hydroxyethylcellulose verdickt sind und mittels PEG niederer Molmasse (200 bis 1000 Dalton) stabilisiert werden können. Die dort offenbarten Produkte sind cremige Massen mit einer Konsistenz, die extrudierbar ist.

Neben der herkömmlichen Handzahnbürste, die vom Verbraucher mit kreisenden Bewegungen im Mund bewegt wird, haben sich elektrische Zahnbürsten im Markt etabliert, die einen Teil der Bewegung der Borsten auf der Zahnoberfläche durch eine im Handstück befindliche - meist wieder aufladbare - Batterie erzeugen. Der Verbraucher kann hierdurch je nach Modell die kreisende Handbewegung durch eine horizontal-lineare Bewegung ersetzen.
Elektrisch bewegte Bürstenköpfe sind dabei in vielfältigen Ausgestaltungen marktüblich. So existieren runde Bürstenköpfe, die elektrisch in Rotation oder Teilrotation mit Drehungen im und gegen den Uhrzeigersinn versetzt werden. Es gibt aber auch länglich, der herkömmlichen Zahnbürste nachempfundene Bürstenköpfe, die elektrisch oszillieren.

Allen elektrischen Zahnbürsten ist der Vorteil gemein, daß sie den Verbraucher anteilsweise von bestimmten Bewegungsabläufen entlasten und im Ruf stehen, die Zähne gründlicher zu reinigen. Diesen Vorteilen stehen aber auch Nachteile gegenüber: Eine elektrische Zahnbürste führt auf der Zahnoberfläche üblicherweise zu einer intensiveren Belastung als beim Zähnputzen per Hand. Werden Art und Gehalt der Schleifkörper in der Zahncreme nicht hierauf abgestimmt, können in Verbindung mit einer elektrischen Zahnbürste Schaden am Zahnschmelz entstehen.

Ein weiteres Problem besteht darin, daß die aufwendige Mechanik im Bürstenkopf verklebt werden kann und die Zahnbürste dann die entsprechende elektrische Bewegung nicht mehr durchführen kann. Zusätzlich muß auch die Rheologie beachtet werden, denn auf den sich bewegenden Bürstenköpfen muß eine bestimmte Haftfähigkeit gegeben sein, um Verspritzen der Zahncreme zu vermeiden.

Auch bei der Verwendung herkömmlicher Zahnbürsten spielt die Rheologie des Zahnreinigungsmittel eine wichtige Rolle: Die Zusammensetzung muß ausreichend viskos sein, um auf den Bürstenkopf appliziert werden zu können, beim Putzen soll sie aber zügig eine Anwendungsmischung im Mundraum ergeben, die eine effektive Plaquereduzierung und eine hohe Fluoriddeposition ermöglicht, was mit niedrigviskosen Zusammensetzungen meist effektiver zu realisieren ist.

Der vorliegenden Erfindung lag die Aufgabe zugrunde, ein Mund- und Zahnpflege- und -reinigungsmittel bereitzustellen, das die vorstehend genannten Aufgabenbereiche, insbesondere die stabile Einarbeitbarkeit von Aminfluoriden, löst und ausreichend viskos ist, um auf Bürstenköpfe herkömmlicher oder elektrischer Zahnbürsten appliziert werden zu können, dabei aber ausreichend niedrigviskos ist, um eine effektive Plaquereduzierung und eine hohe Fluoriddeposition zu ermöglichen.

Es wurde nun gefunden, daß sich niedrigviskose Flüssigzahncremes mit Aminfluoriden formulieren lassen, wenn bestimmte Mengen an Sorbitol eingesetzt und mit mittelgewichtigen Polyethylenglycolen enger Molmassenverteilung stabilisiert werden.

Gegenstand der vorliegenden Erfindung ist in einer ersten Ausführungsform ein Mund- und Zahnpflege- und -reinigungsmittel mit einer Viskosität von 10.000 bis maximal 70.000 mPa·s bei 20°C und 1013,25 mbar (Brookfield Rotationsviskosimeter, Typ RTV, Spindel 5, 2 UpM), enthaltend - bezogen auf sein Gewicht -
a) mindestens 25 Gew.-% Sorbitol, berechnet als 100%ige Aktivsubstanz;
b) 0,1 bis 5 Gew.-% mindestens eines Polyethylenglycols der Formel (I)

   H-[O-CH₂CH₂]ₙ-OH (I),

   in der n für Zahlen zwischen 30 und 40 steht;
c) 1 bis 2,5 Gew.-% mindestens eines Aminfluorids.

Die erfindungsgemäßen Mund- und Zahnpflege- und -reinigungsmittel enthalten als ersten wesentlichen Inhaltsstoff Sorbitol. Dieses fungiert als Matrix für die übrigen Inhaltsstoffe sowie als Feuchthaltemittel und verhindert das Austrocknen der Flüssigzahncreme.
Hier sind erfindungsgemäße Mund- und Zahnpflege- und -reinigungsmittel bevorzugt, die - bezogen auf sein Gewicht - 30 bis 80 Gew.-%, vorzugsweise 32,5 bis 75 Gew.-%, besonders bevorzugt 35 bis 70 Gew.-%, weiter bevorzugt 37,5 bis 65 Gew.-% und insbesondere 40 bis 60 Gew.-% Sorbitol enthalten.

Neben den genannten Mengen an Sorbitol können die erfindungsgemäßen Zusammensetzungen weitere Feuchthaltemittel enthalten, wobei sich Glycerin bzw. 1,2-Propylenglycol besonders bewährt haben. Es ist aber im Hinblick auf die Stabilität der Formulierungen bevorzugt, die Gesamtmenge an Glycerin bzw. 1,2-Propylenglycol kleiner zu halten als die Menge an Sorbitol. Besonders bevorzugte erfindungsgemäße Mittel enthalten weniger als 20 Gew.-%, vorzugsweise weniger als 15 Gew.-%, weiter bevorzugt weniger als 10 Gew.-%, noch weiter bevorzugt weniger als 5 Gew.-% und insbesondere weniger als 1 Gew.-% an Stoffen aus der Gruppe Glycerin bzw. 1,2-Propylenglycol. Ganz besonders bevorzugte Mittel sind frei von Glycerin bzw. 1,2-Propylenglycol.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden.

Besonders bevorzugt sind erfindungsgemäße Mittel, die außer Sorbit keine anderen mehrwertigen Alkohole enthalten.

Die erfindungsgemäßen Mund- und Zahnpflege- und -reinigungsmittel enthalten als zweiten wesentlichen Inhaltsstoff 0,1 bis 5 Gew.-% mindestens eines Polyethylenglycols der Formel (I)

H-[O-CH₂CH₂]ₙ-OH (I),

in der n für Zahlen zwischen 30 und 40 steht;
Diese Polyethylenglycole mit Molmassen zwischen 1338 (n = 30) und 1778 (n = 40) stabilisieren die Aminfluoride auch in niedrigviskosen Sorbitol-basierten Matrices. Vorzugsweise werden Polyethylenglycole einer engeren Molmassenverteilung eingesetzt, wobei bevorzugte erfindungsgemäße Mund- und Zahnpflege- und -reinigungsmittel gemäß Anspruch dadurch gekennzeichnet sind, daß sie - bezogen auf ihr Gewicht - 0,2 bis 4 Gew.-%, vorzugsweise 0,3 bis 3,5 Gew.-%, besonders bevorzugt 0,4 bis 3 Gew.-%, weiter bevorzugt 0,5 bis 2,5 Gew.-% und insbesondere 1 bis 2 Gew.-% mindestens eines Polyethylenglycols der Formel (I) enthalten, in der n für Zahlen zwischen 31 und 39, vorzugsweise zwischen 32 und 38, weiter bevorzugt zwischen 33 und 37 und insbesondere zwischen 34 und 36 steht.

Ganz besonders bevorzugte erfindungsgemäße Mittel enthalten demnach - bezogen auf ihr Gewicht - 0,2 bis 4 Gew.-%, vorzugsweise 0,3 bis 3,5 Gew.-%, besonders bevorzugt 0,4 bis 3 Gew.-%, weiter bevorzugt 0,5 bis 2,5 Gew.-% und insbesondere 1 bis 2 Gew.-% Verbindung(en) aus der Gruppe H-[O-CH₂CH₂]₃₄-OH (MG 1514 gmol⁻¹), H-[O-CH₂CH₂]₃₅-OH (MG 1558 gmol⁻¹), H-[O-CH₂CH₂]₃₆-OH (MG 1602 gmol⁻¹).

Die erfindungsgemäßen Mund- und Zahnpflege- und -reinigungsmittel enthalten als dritten wesentlichen Inhaltsstoff 1 bis 2,5 Gew.-% mindestens eines Aminfluorids.

Als Aminfluoride werden die Hydrofluoride von Aminen bezeichnet. Die Stoffgruppenbezeichnung "Aminfluorid" ist gebräuchlich, chemisch aber nicht korrekt.

Erfindungsgemäß bevorzugte Aminfluoride sind Ethanolamin-Hydrofluorid, Olaflur (ein Dihydrofluorid), Oleaflur und Dectaflur (ein Monohydrofluorid). Vorzugsweise werden die Stoffe aus der Gruppe der Aminfluoride innerhalb engerer Mengenbereiche eingesetzt, so daß erfindungsgemäß besonders bevorzugte Mund- und Zahnpflege- und -reinigungsmittel- bezogen auf ihr Gewicht - 1,25 bis 2,25 Gew.-%, vorzugsweise 1,5 bis 2,0 Gew.-% und insbesondere 1,7 bis 1,9 Gew.-% Aminfluorid(e) enthalten.

Ein besonders bevorzugt einsetzbares Aminfluorid ist Olaflur (Intentaionaler Freiname für *N,N,N'-*Tris(2-hydroxyethyl)-*N*'-octadecyl-1,3-diaminopropandihydrofluorid, auch *N*-(*N,N*-Bis-(hydroxyethyl)-aminopropyl)-*N*-hydroxyethyl-octadecylamin-dihydrofluorid). Besonders bevorzugte erfindungsgemäße Mund- und Zahnpflege- und -reinigungsmittel sind dadurch gekennzeichnet, daß sie - bezogen auf ihr Gewicht - 1,25 bis 2,25 Gew.-%, vorzugsweise 1,5 bis 2,0 Gew.-% und insbesondere 1,7 bis 1,9 Gew.-% *N,N,N'*-Tris(2-hydroxyethyl)- *N'*-octadecyl-1,3- diaminopropandihydrofluorid enthalten.

Zusätzlich zu dem bzw. den Aminfluoriden können die erfindungsgemäßen Mittel weitere Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein. Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthalten, sind erfindungsgemäß bevorzugt.

Besonders bevorzugte erfindungsgemäße Mund- und Zahnpflege- und -reinigungsmittel enthalten zusätzlich Natriumfluorid.

Bei der letztgenannten bevorzugten Ausführungsform sind erfindungsgemäße Mittel bevorzugt , bei denen das Gewichtsverhältnis von Aminfluorid(en) zu Natriumfluorid 100:1 bis 1:10, vorzugsweise 50:1 bis 1:5, weiter bevorzugt 25:1 bis 1:2 und insbesondere 15:1 bis 1:1 beträgt.

Durch die erfindungsgemäße Kombination aus hohem Sorbitolgehalt und Einsatz von Polyethylenglycolen einer definierten und engen Molmassenverteilung ist es möglich, Aminfluoride auch in niedrigviskosen Zusammensetzungen ausreichend zu stabilisieren und so die Vorteile des Einsatzes von Aminfluoriden auch jenseits der pastösen hochviskosen Zusammensetzungen zugänglich zu machen. Dies ermöglicht einerseits die Vorteile von Flüssigzahncremes, beispielsweise die verbesserte Verträglichkeit mit elektrischen Zahnbürsten, andererseits ist die Deposition von Animfluorid aus der niedrigviskosen Matrix weiter verbessert.

Vorzugsweise liegen auch die Viskositäten der erfindungsgemäßen Mittel innerhalb engerer Bereiche. Bevorzugte Mund- und Zahnpflege- und -reinigungsmittel sind dadurch gekennzeichnet, daß sie eine Viskosität (gemessen bei 20°C und 1013,25 mbar, Brookfield Rotationsviskosimeter, Typ RTV, Spindel 5, 2 UpM) von 12.500 bis 60.000 mPas, vorzugsweise von 15.000 bis 50.000 mPas, weiter bevorzugt von 17.500 bis 40.000 mPas und insbesondere von 20.000 bis 37.500 mPas aufweisen.

Je nach Gehalt an Sorbitol, PEG und Wasser kann die Viskosität der erfindungsgemäßen Mittel variieren. Sofern die gewünschten Einsatzmengen dieser drei Stoffe noch nicht in den gewünschten Viskositätsbereich führen, ist es möglich, die erfindungsgemäßen Zusammensetzungen zu verdicken. Dabei haben sich einige Verdicker als besonders verträglich mit der erfindungsgemäßen Kombination erwiesen.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon. Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol-Copolymere mit einem mittleren Molgewicht von 1000 bis 5000 oder eine Kombination der genannten Verbindungen in den Zahnpasten verwendet werden.
Erfindungsgemäß bevorzugte Mund- und Zahnpflege- und -reinigungsmittel enthalten - bezogen auf ihr Gewicht - 0,1 bis 2 Gew.-%, vorzugsweise 0,25 bis 1,5 Gew.-% und insbesondere 0,5 bis 1 Gew.-% mindestens eines Emulgators mit mindestens 40 EO-Gruppen im Molekül.

Ein besonders bevorzugter Verdicker ist die Hydroxyethylcellulose. Erfindungsgemäß bevorzugte Mund- und Zahnpflege- und -reinigungsmittel sind dadurch gekennzeichnet, daß sie zusätzlich - bezogen auf ihr Gewicht - 0,1 bis 2 Gew.-%, vorzugsweise 0,25 bis 1,5 Gew.-%, weiter bevorzugt 0,4 bis 1 Gew.-% und insbesondere 0,5 bis 0,75 Gew.-% Hydroxyethylcellulose enthalten.

Die erfindungsgemäßen Mittel können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mund- und Zahnpflege- und - reinigungsmittel sind dadurch gekennzeichnet, daß sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthalten. Die erfindungsgemäßen Mittel können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.
Der Einsatz von Putzkörpern (Abrasiva) ist ebenfalls bevorzugt. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel).

Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident®12 DS (DEGUSSA) erhältlich.

Andere Fällungskieselsäuren dieser Art sind Sident® 8 (DEGUSSA) und Sorbosil® AC 39 (Crosfield Chemicals). Diese Kieselsäuren zeichnen sich durch eine geringere Verdickungswirkung und eine etwas höhere mittlere Teilchengröße von 8 - 14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungskieselsäuren mit einer BET-Oberfläche von 150 -250 m²/g zu, z.B. die Handelsprodukte Sipernat 22 LS oder Sipernat® 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind dadurch gekennzeichnet, daß sie 0,25 bis 2 Gew.-%, vorzugsweise 0,5 bis 1,5 Gew.-% und insbesondere 0,75 bis 1,25 Gew.-% Aluminiumoxid enthalten.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Mund- und Zahnpflege- und reinigungsmittel können z.B. auch Substanzen enthalten, die gegen Plaque und/oder Zahnstein wirksam sind.
Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.
Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Auch oberflächenaktive Substanzen sind in den erfindungsgemäßen Mitteln einsetzbar. Sie dienen beispielsweise in Zahnpasten zur Unterstützung der Reinigungswirkung und gewünschtenfalls auch zur Entwicklung von Schaum beim Zähnebürsten oder beim Mundspülen sowie zur Stabilisierung der Polierkörperdispersion im Träger und werden sowohl in Mundspüllösungen als auch in Zahnpasten üblicherweise in einer Menge von 0,1-5 Gew.-% eingesetzt.
Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und - diglyceriden, von Fettsäure-Sorbitanestern und Alkyl(oligo)-Glucoside sowie Fettsäureamidoalkylbetaine.

Eine bevorzugt einzusetzende Gruppe von Tensiden stellen die anionischen Tenside dar. Erfindungsgemäß bevorzugte Mund und Zahnpflege- und -reinigungsmittel sind dadurch gekennzeichnet, daß sie 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,6 bis 2,2 Gew.-% anionische(s) Tensid(e) enthalten.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid-(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)-phosphate. Sofern die anionischen Tenside Polyglycoletherketten enthalten, können diese eine konventionelle, vorzugsweise jedoch eine eingeengte Homologenverteilung aufweisen.

Ganz besonders bevorzugte erfindungsgemäße Mittel enthalten Alkylsulfat(e) als anionisches Tensid. Hier sind erfindungsgemäß besonders bevorzugte Mund und Zahnpflege- und -reinigungsmittel sind dadurch gekennzeichnet, daß sie 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,6 bis 2,2 Gew.-% Natriumdodecylsulfat enthalten.

Mit besonderem Vorzug enthalten die erfindungsgemäßen Mittel zusätzlich oder alternativ zu den anionischen Tensiden amphotere(s) Tensid(e). Unter ampholytischen Tensiden und Emulgatoren werden solche oberflächenaktiven Verbindungen verstanden, die außer einer C₈-C₂₄-Alkyl- oder-Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂-C₁₈-Acylsarcosin.

Besonders bevorzugte erfindungsgemäße Mund und Zahnpflege- und -reinigungsmittel sind dadurch gekennzeichnet, daß sie es 0,01 bis 2 Gew.-%, vorzugsweise 0,05 bis 1,5 Gew.-%, weiter bevorzugt 0,1 bis 1 Gew.-%, noch weiter bevorzugt 0,12 bis 0,7 Gew.-% und insbesondere 0,15 bis 0,6 Gew.-% amphotere(s) Tensid(e) enthalten.

Besonders bevorzugte erfindungsgemäße Mund und Zahnpflege- und -reinigungsmittel sind dadurch gekennzeichnet, daß sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂-C₁₈-Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen
enthalten.

Besonders bevorzugte Mund und Zahnpflege- und -reinigungsmittel enthalten als amphotere Tenside Betaine der Formel (Bet-I) 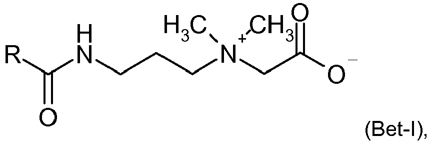 in der R für einen geradkettigen oder verzweigten, gesättigten oder ein- bzw. mehrfach ungesättigten Alkyl- oder Alkenylrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:

H₃C-(CH₂)₇-C(O)-NH-(CH₂)₃N⁺(CH₃)₂CH₂COO⁻

H₃C-(CH₂)₉-C(O)-NH-(CH₂)₃N⁺(CH₃)₂CH₂COO⁻

H₃C-(CH₂)₁₁-C(O)-NH-(CH₂)₃N⁺(CH₃)₂CH₂COO⁻

H₃C-(CH₂)₁₃-C(O)-NH-(CH₂)₃N⁺(CH₃)₂CH₂COO⁻

H₃C-(CH₂)₁₅-C(O)-NH-(CH₂)₃N⁺(CH₃)₂CH₂COO⁻

H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb engerer Mengenbereiche eingesetzt. Hier sind erfindungsgemäße Mund und Zahnpflege- und -reinigungsmittel bevorzugt, die - bezogen auf ihr Gewicht - 0,01 bis 2 Gew.-%, vorzugsweise 0,05 bis 1,5 Gew.-%, weiter bevorzugt 0,1 bis 1 Gew.-%, noch weiter bevorzugt 0,12 bis 0,7 Gew.-% und insbesondere 0,15 bis 0,6 Gew.-% Cocoamidopropylbetaine enthalten.

Erfindungsgemäß beträgt das Gewichtsverhältnis von Poliermittel(n) zu Tensid(en) bevorzugt ≤ 6, d.h. die Poliermittel werden maximal im 6-fachen (Gewichts-)Überschuß zu den Tensiden eingesetzt. Das Gewichtsverhältnis bezieht sich dabei auf das Gewichtsverhältnis aller Stoffe aus den genannten Gruppen zueinander, wobei nur die Aktivsubstanzen gerechnet werden. Enthält ein erfindungsgemäßes Mittel z.B. 12 Gew.-% Poliermittel und 4 Gew.-% einer 50%igen Tensidlösung, so beträgt das Gewichtsverhältnis 12 / 2 = 6. Enthält ein erfindungsgemäßes Mittel z.B. 12 Gew.-% Poliermittel und 4 Gew.-% einer 50%igen Tensidlösung sowie 1 Gew.-% reines Natriumdodecylsulfat, so beträgt das Gewichtsverhältnis 12 / (2 + 1) = 4.

In erfindungsgemäß bevorzugten Mitteln liegt das Gewichtsverhältnis innerhalb eines noch engeren Bereiches. Besonders bevorzugte Mund und Zahnpflege- und -reinigungsmittel sind dadurch gekennzeichnet, daß das Gewichtsverhältnis von Poliermittel(n) zu Tensid(en) im Bereich ≥ 5 bis ≤ 15, vorzugsweise im Bereich ≥ 7,5 bis ≤ 12,5, weiter bevorzugt im Bereich ≥ 10 bis ≤ 12 und insbesondere im Bereich ≥ 10,25 bis ≤ 11,9 liegt.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.
Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten und Mundwässer oder Mundspüllösungen sind
- Oberflächenaktive Stoffe, bevorzugt anionische, zwitterionische, amphotere, nicht- ionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol
- Pigmente, wie z.B. Titandioxid
- Farbstoffe
- Puffersubstanzen, z.B. primäre, sekundäre oder tertiäre Alkaliphosphate oder Citronen- säure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Es hat sich gezeigt, daß die erfindungsgemäßen Mund- und Zahnpflege- und -reinigungsmittel in ihrer Leistung weiter gesteigert werden können, wenn die Mittel salivationsfördernde Substanzen enthalten. Insbesondere die antibakterielle Wirkung und mit ihr die Antikarieswirkung und die Wirkung gegen Gingivitis und/oder Parodontitis werden hierdurch verstärkt.

Unter Salivation versteht man die Speichelproduktion und -freisetzung, im weiteren Sinne auch in unphysiologisch erhöhter Menge. Substanzen, die den Speichelfluß anregen und die Speichelmenge und/oder -freisetzung erhöhen, können aus den unterschiedlichsten Stoffklassen stammen.

Weitere salivationsfördernde Substanzen sind insbesondere sogenannte Scharfstoffe, d.h. scharf schmeckende und/oder ein Gefühl von Wärme erzeugende Substanzen. Erfindungsgemäß bevorzugte Mund- und Zahnpflege- und -reinigungsmittel sind dadurch gekennzeichnet, daß sie als salivationsfördernde Substanz mindestens eine scharf schmeckende und/oder ein Gefühl von Wärme erzeugende Substanz enthalten.

Als salivationsfördernden Inhaltsstoff enthalten die erfindungsgemäßen Erzeugnisse dieser Ausführungsform eine scharf schmeckende und/oder ein Gefühl von Wärme erzeugende Substanz. Diese Substanzen vermitteln dem Anwender einen scharfen, kribbelnden, mundwässernden oder wärmeerzeugenden Effekt, d.h. sie rufen sensorisch einen Wärmeeindruck oder ein Brennen, oder ein Prickeln, Perlen, Kitzeln oder Sprudeln hervor und fördern dadurch den Speichelfluß.

Erfindungsgemäß bevorzugte Erzeugnisse dieser Ausführungsform enthalten die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,75 Gew.-% und insbesondere von 0,01 bis 0,5 Gew.-%, jeweils bezogen auf das Gewichts des gesamten Mittels.

Ein weiterer Gegenstand der vorliegenden Erfindung ist die Verwendung von erfindungsgemäßen Mitteln zur Reinigung von Zähnen mittels elektrischer Zahnbürsten.

Ein weiterer Gegenstand der vorliegenden Erfindung ist ein Verfahren zum Reinigen von Zähnen, dadurch gekennzeichnet, daß ein erfindungsgemäßes Mittel auf den Bürstenkopf einer elektrischen Zahnbürste aufgetragen und mit der elektrischen Zahnbürste die Zähne geputzt werden.

Ein weiterer Gegenstand der vorliegenden Erfindung ist ein Verfahren zur Zahnreinigung, gekennzeichnet durch die Schritte
a) Bereitstellung einer Zahnbürste, deren Bürstenkopf elektrisch in Bewegung versetzt werden kann;
b) Applikation von 0,5 bis 5 g eines erfindungsgemäßen Mittels auf den Bürstenkopf,
c) 30- bis 300-sekündiges Zähneputzen mit dem erfindungsgemäßen Mittel unter Einsatz des elektrisch in Bewegung versetzten Bürstenkopfes.

Bezüglich bevorzugter Ausführungsformen der erfindungsgemäßen Verwendung und der erfindungsgemäßen Verfahren gilt mutatis mutandis das zu den erfindungsgemäßen Mitteln Gesagte.

### Beispiele:

**Alle Angaben in Gew.-%**

| | **A** | **B** | **C** | **D** | **E** | **F** |
|---|---|---|---|---|---|---|
| Sorbitol 70%ig | 19,58 | 28,23 | 23,18 | 19,58 | 28,23 | 23,18 |
| Polyethylenglycol MG 1500 | 1,0 | 1,5 | 1,5 | 1,0 | 1,5 | 1,5 |
| Olaflur | 5,37 | 5,37 | 5,37 | 2,70 | 2,70 | 2,70 |
| Natriumfluorid | 0 | 0 | 0 | 0,16 | 0,16 | 0,16 |
| Hydroxyethylcellulose | 0,7 | 0,55 | 0,6 | 0,7 | 0,55 | 0,6 |
| 1,2-Propandiol | 5,0 | 0 | 0 | 5,0 | 0 | 0 |
| Sident 8 | 12 | 12 | 12 | 12 | 12 | 12 |
| Titanium Dioxide | 1 | 1 | 1 | 1 | 1 | 1 |
| Saccharin Natrium | 0,25 | 0,25 | 0,25 | 0,25 | 0,25 | 0,25 |
| Sident 22 S | 0 | 0 | 0 | 9,0 | 9,0 | 9,0 |
| Calciumglycerophosphat | 0 | 0 | 0 | 0,13 | 0,2 | 0,2 |
| Dinatriumphosphat wasserfrei | 0 | 0 | 0 | 0,2 | 0,2 | 0,2 |
| Trinatriumphosphat wasserfrei | 0 | 0 | 0 | 0,3 | 0,3 | 0,3 |
| Natriumlaurylsulfat | 0 | 0 | 0 | 1,0 | 1,2 | 1,0 |
| Aroma | 0,1 | 0,2 | 0,3 | 0,1 | 0,2 | 0,3 |
| Wasser | ad 100 | ad 100 | ad 100 | ad 100 | ad 100 | ad 100 |
| | | | | | | |
| Viskosität * [mPas] | 35.600 | 22.500 | 27.150 | 41.200 | 35.800 | 38.200 |
| Stabiltät ** | | | | | | |
| nach Herstellung | 1 | 1 | 1 | 1 | 1 | 1 |
| nach 6 Monaten Lagerung bei 20°C | 1 | 1 | 1 | 1 | 1 | 1 |

| | | | | | | |
|---|---|---|---|---|---|---|
| * bei 20°C und 1013,25 mbar (Brookfield Rotationsviskosimeter, Typ RTV, Spindel 5, 2 UpM ** Schulnotensystem: 1 keine Trennung 2 leichte Trübung 3 mittlere Trübung 4 schwere Trübung 5 schwere Trübung, Absetzen 6 Ausflocken und Absetzen | | | | | | |

## Patentansprüche

1. Mund- und Zahnpflege- und -reinigungsmittel mit einer Viskosität von 10.000 bis maximal 70.000 mPa·s bei 20°C und 1013,25 mbar (Brookfield Rotationsviskosimeter, Typ RTV, Spindel 5, 2 UpM), enthaltend - bezogen auf sein Gewicht -
a) mindestens 25 Gew.-% Sorbitol, berechnet als 100%ige Aktivsubstanz;
b) 0,1 bis 5 Gew.-% mindestens eines Polyethylenglycols der Formel (I)
H-[O-CH₂CH₂]ₙ-OH (I),
in der n für Zahlen zwischen 30 und 40 steht;
c) 1 bis 2,5 Gew.-% mindestens eines Aminfluorids.

2. Mund- und Zahnpflege- und -reinigungsmittel gemäß Anspruch 1, **dadurch gekennzeichnet, daß** es - bezogen auf sein Gewicht - 30 bis 80 Gew.-%, vorzugsweise 32,5 bis 75 Gew.-%, besonders bevorzugt 35 bis 70 Gew.-%, weiter bevorzugt 37,5 bis 65 Gew.-% und insbesondere 40 bis 60 Gew.-% Sorbitol enthält.

3. Mund- und Zahnpflege- und -reinigungsmittel gemäß Anspruch 1 oder 2, **dadurch gekennzeichnet, daß** es - bezogen auf sein Gewicht - 0,2 bis 4 Gew.-%, vorzugsweise 0,3 bis 3,5 Gew.-%, besonders bevorzugt 0,4 bis 3 Gew.-%, weiter bevorzugt 0,5 bis 2,5 Gew.-% und insbesondere 1 bis 2 Gew.-% mindestens eines Polyethylenglycols der Formel (I) enthält, in der n für Zahlen zwischen 31 und 39, vorzugsweise zwischen 32 und 38, weiter bevorzugt zwischen 33 und 37 und insbesondere zwischen 34 und 36 steht.

4. Mund- und Zahnpflege- und -reinigungsmittel gemäß einem der Ansprüche 1 bis 3, **dadurch gekennzeichnet, daß** es - bezogen auf sein Gewicht - 1,25 bis 2,25 Gew.-%, vorzugsweise 1,5 bis 2,0 Gew.-% und insbesondere 1,7 bis 1,9 Gew.-% Aminfluorid(e) enthält.

5. Mund- und Zahnpflege- und -reinigungsmittel nach einem der Ansprüche 1 bis 4, **dadurch gekennzeichnet, daß** es es - bezogen auf sein Gewicht - 1,25 bis 2,25 Gew.-%, vorzugsweise 1,5 bis 2,0 Gew.-% und insbesondere 1,7 bis 1,9 Gew.-% *N,N,N'*-Tris(2-hydroxyethyl)-*N*'-octadecyl-1,3-diaminopropan-dihydrofluorid enthält.

6. Mund- und Zahnpflege- und -reinigungsmittel nach einem der Ansprüche 1 bis 5, **dadurch gekennzeichnet, daß** es zusätzlich Natriumfluorid enthält.

7. Mund- und Zahnpflege- und -reinigungsmittel nach Anspruch 6, **dadurch gekennzeichnet, daß** das Gewichtsverhältnis von Aminfluorid(en) zu Natriumfluorid 100:1 bis 1:10, vorzugsweise 50:1 bis 1:5, weiter bevorzugt 25:1 bis 1:2 und insbesondere 15:1 bis 1:1 beträgt.

8. Mund- und Zahnpflege- und -reinigungsmittel nach einem der Ansprüche 1 bis 7, **dadurch gekennzeichnet, daß** es zusätzlich - bezogen auf sein Gewicht - 0,1 bis 2 Gew.-%, vorzugsweise 0,25 bis 1,5 Gew.-% und insbesondere 0,5 bis 1 Gew.-% mindestens eines Emulgators mit mindestens 40 EO-Gruppen im Molekül enthält.

9. Mund- und Zahnpflege- und -reinigungsmittel nach einem der Ansprüche 1 bis 8, **dadurch gekennzeichnet, daß** es eine Viskosität (gemessen bei 20°C und 1013,25 mbar, Brookfield Rotationsviskosimeter, Typ RTV, Spindel 5, 2 UpM) von 12.500 bis 60.000 mPas, vorzugsweise von 15.000 bis 50.000 mPas, weiter bevorzugt von 17.500 bis 40.000 mPas und insbesondere von 20.000 bis 37.500 mPas aufweist.

10. Mund- und Zahnpflege- und -reinigungsmittel nach einem der Ansprüche 1 bis 9, **dadurch gekennzeichnet, daß** es zusätzlich - bezogen auf sein Gewicht - 0,1 bis 2 Gew.-%, vorzugsweise 0,25 bis 1,5 Gew.-%, weiter bevorzugt 0,4 bis 1 Gew.-% und insbesondere 0,5 bis 0,75 Gew.-% Hydroxyethylcellulose enthält.

11. Mund und Zahnpflege- und -reinigungsmittel nach einem der Ansprüche 1 bis 10, **dadurch gekennzeichnet, daß** es 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,6 bis 2,2 Gew.-% anionische(s) Tensid(e) enthält.

12. Mund und Zahnpflege- und -reinigungsmittel nach einem der Ansprüche 1 bis 11, **dadurch gekennzeichnet, daß** es 0,25 bis 4 Gew.-%, vorzugsweise 0,5 bis 3,5 Gew.-%, weiter bevorzugt 0,75 bis 3 Gew.-%, noch weiter bevorzugt 1 bis 2,5 Gew.-% und insbesondere 1,6 bis 2,2 Gew.-% Natriumdodecylsulfat enthält.

13. Zahnpflege- und -reinigungsmittel nach einem der Ansprüche 1 bis 12, **dadurch gekennzeichnet, daß** es 0,01 bis 2 Gew.-%, vorzugsweise 0,05 bis 1,5 Gew.-%, weiter bevorzugt 0,1 bis 1 Gew.-%, noch weiter bevorzugt 0,12 bis 0,7 Gew.-% und insbesondere 0,15 bis 0,6 Gew.-% amphotere(s) Tensid(e) enthält.

14. Mund und Zahnpflege- und -reinigungsmittel nach einem der Ansprüche 1 bis 13, **dadurch gekennzeichnet, daß** es - bezogen auf sein Gewicht - 0,01 bis 2 Gew.-%, vorzugsweise 0,05 bis 1,5 Gew.-%, weiter bevorzugt 0,1 bis 1 Gew.-%, noch weiter bevorzugt 0,12 bis 0,7 Gew.-% und insbesondere 0,15 bis 0,6 Gew.-% Cocoamidopropylbetaine enthält.

15. Mund und Zahnpflege- und -reinigungsmittel nach einem der Ansprüche 1 bis 14, **dadurch gekennzeichnet, daß** das Gewichtsverhältnis von Poliermittel(n) zu Tensid(en) im Bereich ≥ 5 bis ≤ 15, vorzugsweise im Bereich ≥ 7,5 bis ≤ 12,5, weiter bevorzugt im Bereich ≥ 10 bis ≤ 12 und insbesondere im Bereich ≥ 10,25 bis ≤ 11,9 liegt.

## Claims

1. An oral and dental care and cleaning agent having a viscosity of from 10,000 to a maximum of 70,000 mPa·s at 20 °C and 1013.25 mbar (Brookfield rotational viscometer, type RTV, spindle 5, 2 rpm), containing, based on its weight:
a) at least 25 wt.% sorbitol, calculated as 100% active substance;
b) 0.1 to 5 wt.% of at least one polyethylene glycol of formula (I)
H-[O-CH₂CH₂]ₙ-OH (I),
in which n represents numbers between 30 and 40;
c) 1 to 2.5 wt.% of at least one amine fluoride.

2. The oral and dental care and cleaning agent according to claim 1, **characterized in that** it contains, based on its weight, 30 to 80 wt.%, preferably 32.5 to 75 wt.%, more preferably 35 to 70 wt.%, particularly preferably 37.5 to 65 wt.%, and in particular 40 to 60 wt.% sorbitol.

3. The oral and dental care and cleaning agent according to claim 1 or 2, **characterized in that** it contains, based on its weight, 0.2 to 4 wt.%, preferably 0.3 to 3.5 wt.%, more preferably 0.4 to 3 wt.%, particularly preferably 0.5 to 2.5 wt.%, and in particular 1 to 2 wt.% of at least one polyethylene glycol of formula (I), in which n represents numbers between 31 and 39, preferably between 32 and 38, particularly preferably between 33 and 37, and in particular between 34 and 36.

4. The oral and dental care and cleaning agent according to one of claims 1 to 3, **characterized in that** it contains, based on its weight, 1.25 to 2.25 wt.%, preferably 1.5 to 2.0 wt.%, and in particular 1.7 to 1.9 wt.% amine fluoride(s).

5. The oral and dental care and cleaning agent according to one of claims 1 to 4, **characterized in that** it contains, based on its weight, 1.25 to 2.25 wt.%, preferably 1.5 to 2.0 wt.%, and in particular 1.7 to 1.9 wt.% *N,N,N*'-tris(2-hydroxyethyl)-*N*'-octadecyl-1,3-diaminopropane-dihydrofluoride.

6. The oral and dental care and cleaning agent according to one of claims 1 to 5, **characterized in that** it additionally contains sodium fluoride.

7. The oral and dental care and cleaning agent according to claim 6, **characterized in that** the weight ratio of amine fluoride(s) to sodium fluoride is 100:1 to 1:10, preferably 50:1 to 1:5, more preferably 25:1 to 1:2, and in particular 15:1 to 1:1.

8. The oral and dental care and cleaning agent according to one of claims 1 to 7, **characterized in that** it additionally contains, based on its weight, 0.1 to 2 wt.%, preferably 0.25 to 1.5 wt.%, and in particular 0.5 to 1 wt.% of at least one emulsifier having at least 40 EO groups in the molecule.

9. The oral and dental care and cleaning agent according to one of claims 1 to 8, **characterized in that** it has a viscosity (measured at 20 °C and 1013.25 mbar, Brookfield rotational viscometer, type RTV, spindle 5, 2 rpm) of from 12,500 to 60,000 mPas, preferably from 15,000 to 50,000 mPas, more preferably from 17,500 to 40,000 mPas, and in particular from 20,000 to 37,500 mPas.

10. The oral and dental care and cleaning agent according to one of claims 1 to 9, **characterized in that** it additionally contains, based on its weight, 0.1 to 2 wt.%, preferably 0.25 to 1.5 % wt.%, more preferably 0.4 to 1 wt.%, and in particular 0.5 to 0.75 wt.% hydroxyethyl cellulose.

11. The oral and dental care and cleaning agent according to one of claims 1 to 10, **characterized in that** it contains 0.25 to 4 wt.%, preferably 0.5 to 3.5 wt.%, more preferably 0.75 to 3 wt.%, even more preferably 1 to 2.5 wt.%, and in particular 1.6 to 2.2 wt.% anionic surfactant(s).

12. The oral and dental care and cleaning agent according to one of claims 1 to 11, **characterized in that** it contains 0.25 to 4 wt.%, preferably 0.5 to 3.5 wt.%, more preferably 0.75 to 3 wt.%, even more preferably 1 to 2.5 wt.%, and in particular 1.6 to 2.2 wt.% sodium dodecyl sulfate.

13. The dental care and cleaning agent according to one of claims 1 to 12, **characterized in that** it contains 0.01 to 2 wt.%, preferably 0.05 to 1.5 wt.%, more preferably 0.1 to 1 wt.%, even more preferably 0.12 to 0.7 wt.%, and in particular 0.15 to 0.6 wt.% amphoteric surfactant(s).

14. The oral and dental care and cleaning agent according to one of claims 1 to 13, **characterized in that** it contains, based on its weight, 0.01 to 2 wt.%, preferably 0.05 to 1.5 wt.%, more preferably 0.1 to 1 wt.%, even more preferably 0.12 to 0.7 wt.%, and in particular 0.15 to 0.6 wt.% cocoamidopropyl betaine.

15. The oral and dental care and cleaning agent according to one of claims 1 to 14, **characterized in that** the weight ratio of polishing agent(s) to surfactant(s) is in the range of from ≥ 5 to ≤ 15, preferably in the range of from ≥ 7.5 to ≤ 12.5, more preferably in the range of from ≥ 10 to ≤ 12, and in particular in the range of from ≥ 10.25 to ≤ 11.9.

## Revendications

1. Agent de nettoyage et de soins bucco-dentaires ayant une viscosité de 10 000 à 70 000 mPa.s maximum à 20 °C et à 1013,25 mbar (Viscosimètre rotatif Brookfield, modèle RTV, broche 5, 2 tours par minute) contenant rapporté à son poids :
a) au moins 25 % en poids de sorbitol, calculé en tant que substance active à 100 % ;
b) 0,1 à 5 % en poids d'au moins un polyéthylène glycol de la formule (I)
H-[O-CH₂CH₂]ₙ-OH (I)
dans laquelle n représente des nombres compris entre 30 et 40 ;
c) 1 à 2,5 % en poids d'au moins un fluorure d'amine.

2. Agent de nettoyage et de soins bucco-dentaires selon la revendication 1, **caractérisé en ce qu'**il contient, sur la base de son poids, de 30 à 80 % en poids, de préférence de 32,5 à 75 % en poids, de façon particulièrement préférée de 35 à 70 % en poids, plus préférablement de 37,5 à 65 % en poids et en particulier de 40 à 60 % en poids de sorbitol.

3. Agent de nettoyage et de soins bucco-dentaires selon la revendication 1 ou 2, **caractérisé en ce qu'**il contient, sur la base de son poids, de 0,2 à 4 % en poids, de préférence de 0,3 à 3,5 % en poids, de manière particulièrement préférée de 0,4 à 3 % en poids, plus préférablement de 0,5 à 2,5 % en poids et en particulier de 1 à 2 % en poids d'au moins un polyéthylène glycol de la formule (I) dans laquelle n est compris entre 31 et 39, de préférence entre 32 et 38, plus préférablement entre 33 et 37 et en particulier entre 34 et 36.

4. Agent de nettoyage et de soins bucco-dentaires selon l'une des revendications 1 à 3, **caractérisé en ce qu'**il contient, sur la base de son poids, de 1,25 à 2,25 % en poids, de préférence de 1,5 à 2,0 % en poids et en particulier de 1,7 à 1,9 % en poids d'un ou de plusieurs fluorures d'amine.

5. Agent de nettoyage et de soins bucco-dentaires selon l'une des revendications 1 à 4, **caractérisé en ce qu'**il contient, sur la base de son poids, de 1,25 à 2,25 % en poids, de préférence de 1,5 à 2,0 % en poids, en particulier de 1,7 à 1,9 % en poids de bis fluorhydrate de *N,N,N'*-tris (2-hydroxyéthyl)-*N*'-octadécyl-1,3-diaminopropane.

6. Agent de nettoyage et de soins bucco-dentaires selon l'une des revendications 1 à 5, **caractérisé en ce qu'**il contient en outre du fluorure de sodium.

7. Agent de nettoyage et de soins bucco-dentaires selon la revendication 6, **caractérisé en ce que** le rapport en poids du ou des fluorures d'amine au fluorure de sodium est de 100:1 à 1:10, de préférence de 50:1 à 1:5, plus préférablement de 25:1 à 1:2 et en particulier 15:1 à 1:1.

8. Agent de nettoyage et de soins bucco-dentaires selon l'une des revendications 1 à 7, **caractérisé en ce qu'**il contient en outre, sur la base de son poids, de 0,1 à 2 % en poids, de préférence de 0,25 à 1,5 % en poids et en particulier de 0,5 à 1 % en poids d'au moins un émulsifiant comportant au moins 40 groupes d'OE dans la molécule.

9. Agent de nettoyage et de soins bucco-dentaires selon l'une des revendications 1 à 8, **caractérisé en ce qu'**il présente une viscosité (mesurée à 20 °C et à 1013,25 mbar, viscosimètre rotatif Brookfield, modèle RTV, broche 5, 2 tours par minute) de 12 500 à 60 000 mPa.s, de préférence de 15 000 à 50 000 mPa.s, plus préférablement de 17 500 à 40 000 mPa.s et en particulier de 20 000 à 37 500 mPa.s.

10. Agent de nettoyage et de soins bucco-dentaires selon l'une des revendications 1 à 9, **caractérisé en ce qu'**il contient en outre, sur la base de son poids, de 0,1 à 2 % en poids, de préférence de 0,25 à 1,5 % en poids, plus préférablement de 0,4 à 1 % en poids et en particulier de 0,5 à 0,75 % en poids d'hydroxyéthylcellulose.

11. Agent de nettoyage et de soins bucco-dentaires selon l'une des revendications 1 à 10, **caractérisé en ce qu'**il contient de 0,25 à 4 % en poids, de préférence de 0,5 à 3,5 % en poids, plus préférablement de 0,75 % à 3 % en poids, encore plus préférablement de 1 à 2,5 % en poids et en particulier de 1,6 à 2,2 % en poids d'un ou de plusieurs tensioactifs anioniques.

12. Agent de nettoyage et de soins bucco-dentaires selon l'une des revendications 1 à 11, **caractérisés en ce qu'**il contient de 0,25 à 4 % en poids, de préférence de 0,5 à 3,5 % en poids, plus préférablement de 0,75 à 3 % en poids, encore plus préférablement de 1 à 2,5 % en poids et en particulier de 1,6 à 2,2 % en poids de dodécylsulfate de sodium.

13. Agent de nettoyage et de soins bucco-dentaires selon l'une des revendications 1 à 12, **caractérisé en ce qu'**il contient de 0,01 à 2 % en poids, de préférence de 0,05 à 1,5 % en poids, plus préférablement de 0,1 à 1 % en poids, encore plus préférablement de 0,12 à 0,7 % en poids et en particulier de 0,15 à 0,6 % en poids d'un ou de plusieurs tensioactifs amphotères.

14. Agent de nettoyage et de soins bucco-dentaires selon l'une des revendications 1 à 13, **caractérisé en ce qu'**il contient, sur la base de son poids, de 0,01 à 2 % en poids, de préférence de 0,05 à 1,5 % en poids, plus préférablement de 0,1 à 1 % en poids, encore plus préférablement de 0,12 à 0,7 % en poids et en particulier de 0,15 à 0,6 % en poids de cocoamidopropylbétaïne.

15. Agent de nettoyage et de soins bucco-dentaires selon l'une des revendications 1 à 14, **caractérisé en ce que** le rapport en poids de l'agent ou des agents de polissage sur le ou les tensioactifs est dans la gamme de ≥ 5 à ≤ 15, de préférence dans la gamme de ≥ 7,5 à ≤ 12,5, plus préférablement dans la gamme de ≥ 10 à ≤ 12 et en particulier dans la gamme de ≥ 10,25 à ≤ 11,9.
